# EUROPEAN PATENT APPLICATION

(11) **EP 4 814 099 A1**
(43) Date of publication of application: **30.09.2026**
(21) Application number: 26167102.8
(22) Date of filing: 24.03.2026
(51) Int. Cl.: C12M 1/40, C12M 1/34, C12P 19/00

(54) **SUGAR LIQUID PRODUCTION SYSTEM**

(30) Priority: 26.03.2025 JP 2025052603
(71) Applicant: Seiko Epson Corporation, Tokyo 160-8801 (JP)
(72) Inventor: SEKI, Yoko, Suwa-shi 392-8502 (JP); YAMASAKI, Sayaka, Suwa-shi 392-8502 (JP); NAKASHIMA, Yoshiki, Suwa-shi 392-8502 (JP); TANAKA, Hideki, Suwa-shi 392-8502 (JP)
(74) Representative: Lewis Silkin LLP

(57) **Abstract**

Provided is a sugar liquid production system including a saccharification tank in which a raw material containing cellulose is reacted with an enzyme to obtain a saccharified liquid containing a sugar liquid and a solid, a discharge line that discharges the saccharified liquid from the saccharification tank, a solid-liquid separation section that separates the solid from the saccharified liquid discharged from the discharge line to obtain the sugar liquid, an enzyme recovery section that separates the enzyme and sugars contained in the sugar liquid and recovers the enzyme, an enzyme return line that returns the enzyme recovered by the enzyme recovery section to the saccharification tank, an enzyme detection section that detects a concentration of the enzyme contained in the saccharified liquid in the saccharification tank, a discharge section that performs a discharge operation of the saccharified liquid from the saccharification tank, and a control section that controls actuation of the discharge section based on a detection result of the enzyme detection section and controls the discharge operation.

## Description

The present application is based on, and claims priority from JP Application Serial Number 2025-052603, filed March 26, 2025, the disclosure of which is hereby incorporated by reference herein in its entirety.

### BACKGROUND

### 1. Technical Field

The present disclosure relates to a sugar liquid production system.

### 2. Related Art

In recent years, products for achieving carbon neutrality and carbon negativity have been demanded. Among them, a method for producing, using an enzyme, sugars such as biomass glucose from a cellulose-based biomass raw material has been attracting attention. Sugars can be raw materials for biomass plastics and biomass ethanol.

For example, JP-A-2006-87319 discloses a saccharification method in which a lignocellulose material and an enzyme are mixed and subjected to a saccharification reaction to produce a saccharification reaction liquid, while unreacted lignocellulose material and the enzyme are recovered from the saccharification reaction liquid and circulated to the saccharification reaction tank.

However, in the related art, sufficient research has not been conducted on the efficiency of enzyme recovery, and the efficiency of enzyme recovery has been poor.

### SUMMARY

A sugar liquid production system according to an application example of the present disclosure includes a saccharification tank in which a raw material containing cellulose is reacted with an enzyme to obtain a saccharified liquid containing a sugar liquid and a solid, a discharge line that discharges the saccharified liquid from the saccharification tank, a solid-liquid separation section that separates the solid from the saccharified liquid discharged from the discharge line to obtain the sugar liquid, an enzyme recovery section that separates the enzyme and sugars contained in the sugar liquid and recovers the enzyme, an enzyme return line that returns the enzyme recovered by the enzyme recovery section to the saccharification tank, an enzyme detection section that detects a concentration of the enzyme contained in the saccharified liquid in the saccharification tank, a discharge section that performs a discharge operation of the saccharified liquid from the saccharification tank, and a control section that controls actuation of the discharge section based on a detection result of the enzyme detection section and controls the discharge operation.

### BRIEF DESCRIPTION OF THE DRAWINGS

FIG. 1 is a configuration diagram schematically showing a sugar liquid production system according to an embodiment of the present disclosure.
FIG. 2 is a block diagram of the sugar liquid production system shown in FIG. 1.
FIG. 3 is an example of a graph in which the vertical axis represents the enzyme concentration detected by the enzyme detection section, and the horizontal axis represents the time (elapsed time) during which the saccharification reaction is performed.

### DESCRIPTION OF EMBODIMENTS

Hereinafter, a sugar liquid production system of the present disclosure will be described in detail based on a preferred embodiment illustrated in the accompanying drawings.

### Embodiments

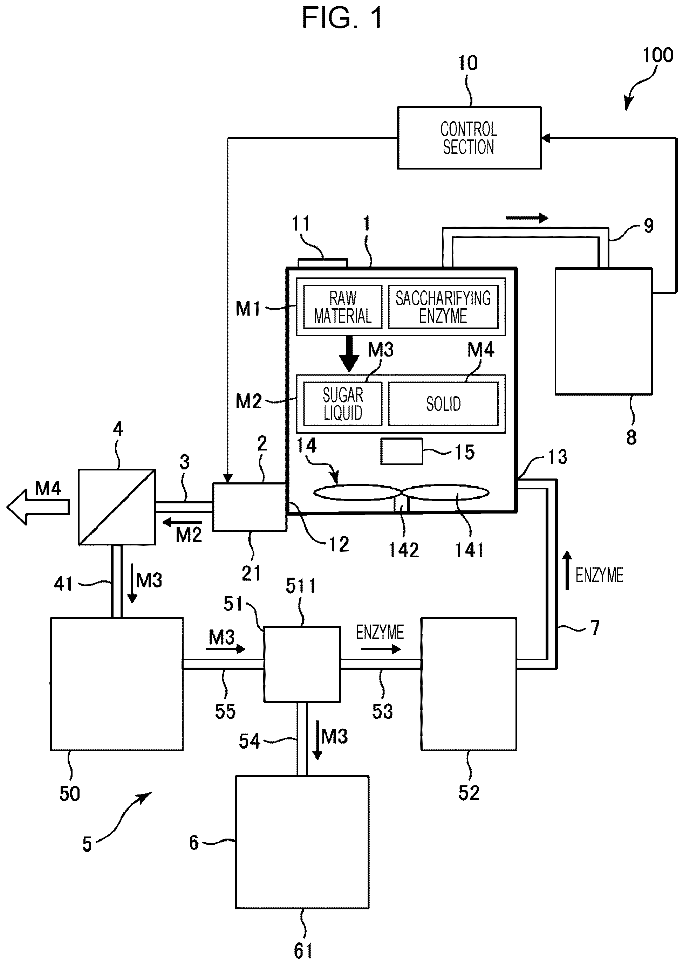

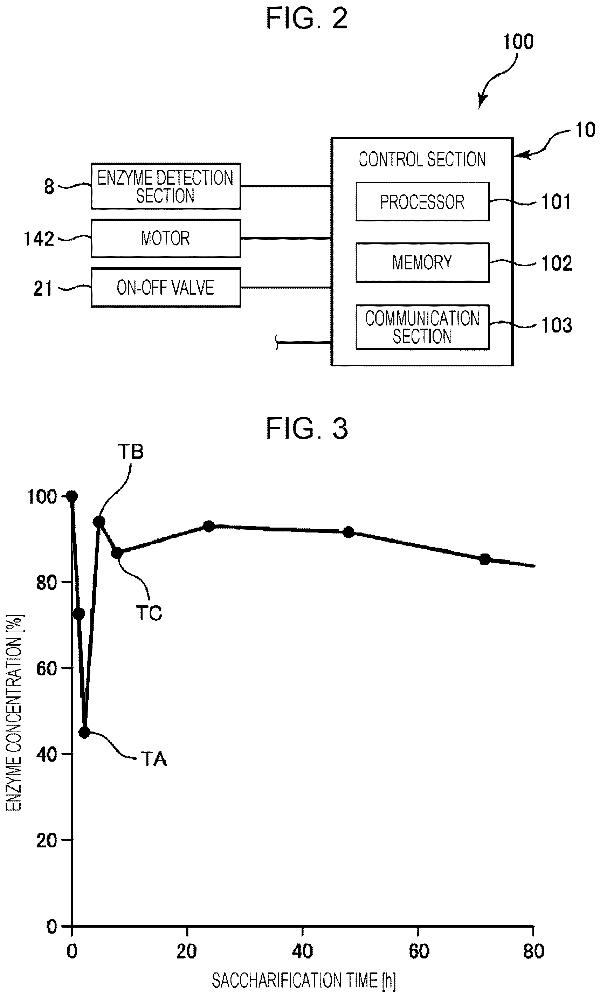

FIG. 1 is a configuration diagram schematically showing a sugar liquid production system according to an embodiment of the present disclosure. FIG. 2 is a block diagram of the sugar liquid production system shown in FIG. 1. FIG. 3 is an example of a graph in which the vertical axis represents the enzyme concentration detected by the enzyme detection section, and the horizontal axis represents the time (elapsed time) during which the saccharification reaction is performed.

As shown in FIG. 1, the sugar liquid production system 100 is an apparatus in which a raw material containing cellulose is reacted with an enzyme to obtain a sugar liquid, and the enzyme is recovered after the reaction and is reused. The sugar liquid production system 100 includes a saccharification tank 1, a discharge section 2, a discharge line 3, a solid-liquid separation section 4, a sugar liquid recovery section 6, an enzyme recovery section 5, an enzyme return line 7, an enzyme detection section 8, and a control section 10. Hereinafter, each section will be described in detail.

### Saccharification Tank 1, Saccharification Reaction Step

As shown in FIG. 1, the saccharification tank 1 is a portion for performing a saccharification reaction step in which a raw material containing cellulose is reacted with an enzyme to obtain a saccharified liquid M2 containing a sugar liquid M3 and a solid M4 (residue). Water, a raw material containing cellulose, and an enzyme are fed into the saccharification tank 1 and stirred in the saccharification tank 1. Hereinafter, the water, raw material, and enzyme fed into the saccharification tank 1 will also be referred to as a "mixed liquid M1". As a result of the reaction between the cellulose and enzyme in the mixed liquid M1 (saccharification reaction), a saccharified liquid M2 containing a sugar liquid M3 and a solid M4 is produced.

### Water

The water is not particularly limited, and examples thereof include pure water such as ion-exchanged water, ultrafiltered water, reverse osmosis water, and distilled water, and ultrapure water with reduced ionic impurities. As a result of using such water, the efficiency of the saccharification reaction can be improved.

As the raw material containing cellulose, not only a pulp-based raw material such as paper but also a herbaceous biomass raw material, a wood-based biomass raw material, or the like can be applied. As the paper to serve as a raw material, not only unused new paper but also printed waste paper can be applied. Examples of printed waste paper include copy paper, newspapers, and magazines. As a result of using printed waste paper, environmental resources and underground resources can be saved, and waste can be reduced.

### Raw Material

The raw material may contain components other than cellulose. Examples of components other than cellulose include wood-derived components such as lignin and hemicellulose, fillers, pigments, resin components, clays, binders, toners, and oils.

The form of cellulose is not particularly limited, and examples thereof include coarsely crushed pieces obtained by a coarse crushing treatment and defibrated materials obtained by a defibration treatment. As a result of such a treatment, components other than cellulose contained in the raw material can be easily liberated from cellulose, and also the efficiency of the saccharification reaction step can be improved.

The raw material has preferably undergone a sterilization treatment. Examples of sterilization treatments include a high-pressure heated steam treatment, an ultraviolet irradiation treatment, and an acid treatment. As a result of a sterilization treatment, glucose and the like generated by the saccharification reaction are less likely to be consumed by microorganisms derived from the raw material and the like, and the efficiency of sugar recovery can be enhanced.

The amount of raw material fed into the saccharification tank 1 is preferably 3% by weight or more and 50% by weight or less, more preferably 5% by weight or more and 20% by weight or less, based on the amount of water fed into the saccharification tank 1. When the amount of raw material fed into the saccharification tank 1 is within the above range, an excessive increase in the viscosity of the mixed liquid M1 can be suppressed, and the efficiency of the saccharification reaction can be further improved.

### Enzyme

The enzyme is not particularly limited as long as it acts to decompose cellulose into sugars by cleaving β-1,4-glucosidic bonds, and examples thereof include endoglucanases, cellobiohydrolases, hemicellulases, and cellobiases (β-glucosidases). One or a combination of two or more of these can be used.

The amount of enzyme (enzyme solution) fed is preferably 0.1% by weight or more and 10% by weight or less, more preferably 2% by weight or more and 5% by weight or less, based on the amount of water fed into the saccharification tank 1. When the amount of raw material fed into the saccharification tank 1 is within the above range, the efficiency of the saccharification reaction can be more effectively enhanced, and also the enzyme can be prevented from being excessively supplied, allowing for cost reduction.

### Other Components

The saccharification tank 1 may be configured such that other components are also fed in addition to the water, raw material, and enzyme. Examples of other components include pH adjusters and surfactants.

Examples of pH adjusters include organic acids such as acetic acid, citric acid, and phosphoric acid, inorganic acids, organic alkalis, and inorganic alkalis, as well as salts thereof, such as sodium salts. One or a combination of two or more of these can be used.

Examples of surfactants include silicone-based defoaming agents, polysiloxane-based defoaming agents, acetylene glycol-based defoaming agents, polyether-based defoaming agents, and fatty acid ester-based defoaming agents. One or a combination of two or more of these can be used.

The saccharification tank 1 has a feed port 11 into which water, a raw material, and an enzyme are fed, a discharge port 12 to which the discharge line 3 described later is connected, a return port 13 to which an enzyme return line 7 is connected, a stirring section 14, and a temperature adjustment section 15. When the above-described saccharification reaction proceeds, a sugar liquid M3 and a solid M4 are produced, and a saccharified liquid M2 containing the sugar liquid M3 and the solid M4 is discharged from the discharge port 12.

As shown in FIG. 1, the stirring section 14 has a stirring blade 141 provided at the bottom of the saccharification tank 1 and a motor 142 that rotates the stirring blade 141. As a result, the mixed liquid M1 in the saccharification tank 1 can be stirred, and the saccharification reaction can be promoted.

The motor 142 is electrically connected to the control section 10, and the rotation speed of the motor 142 is controlled by the control section 10 controlling the energization conditions for the motor 142. As a result, the rotation speed of the stirring blade 141 can be adjusted, and the reaction rate of the saccharification reaction and the reaction efficiency can be adjusted.

The installation position of the stirring blade 141 is not limited to the above. In addition, the stirring section 14 may be omitted.

As shown in FIG. 1, the temperature adjustment section 15 has a heater provided in the saccharification tank 1. The heater is electrically connected to the control section 10, and the temperature of the mixed liquid M1 in the saccharification tank 1 can be adjusted by the control section 10 controlling the energization conditions for the heater.

The heating temperature of the heater is preferably 30°C or more and 60°C or less, and more preferably 40°C or more and 57°C or less. As a result, the enzyme is further activated, and the reaction efficiency can be further improved.

### Discharge Section 2, Discharge Line 3, Discharge Step

As shown in FIG. 1, the discharge section 2 has an on-off valve 21 provided at the discharge port 12. The on-off valve 21 is composed of, for example, an electromagnetic valve. The on-off valve 21 is electrically connected to the control section 10, and the actuation thereof is controlled. The control section 10 can discharge the saccharified liquid M2 from the discharge port 12 by opening the on-off valve 21 at a desired timing. The control section 10 can adjust the discharge amount of the saccharified liquid M2 by closing the on-off valve 21 at a desired timing.

The on-off valve 21 is not limited to the configuration of switching between the open state and the closed state, and may be configured to adjust the opening degree continuously or stepwise.

The saccharified liquid M2 discharged from the discharge port 12 is discharged to the solid-liquid separation section 4 via the discharge line 3. The discharge line 3 may be a flexible tube or a rigid pipe. One end of the discharge line 3 is connected to the discharge port 12, and the other end is connected to the solid-liquid separation section 4.

In addition, the on-off valve 21 may be provided in the discharge line 3.

In addition, a plurality of discharge lines 3 may be provided.

### Solid-Liquid Separation Section 4, Solid-Liquid Separation Step

The solid-liquid separation section 4 has a function of separating a solid M4 from the saccharified liquid M2 discharged from the discharge line 3 to obtain a sugar liquid M3. The separation method of the solid-liquid separation section 4 is not particularly limited, and examples thereof include a filtration separation method, a sedimentation separation method, an extraction separation method, and a centrifugal separation method.

Examples of the solid M4 (residue) include impurities attached to cellulose, such as synthetic resins and metals.

The solid M4 separated in the solid-liquid separation section 4 is discarded, and the sugar liquid M3 separated in the solid-liquid separation section 4 is supplied to the enzyme recovery section 5 via the liquid feed line 41. The sugar liquid M3 contains an enzyme and sugars.

### Enzyme Recovery Section 5, Enzyme Recovery Step

As shown in FIG. 1, the enzyme recovery section 5 has a function of separating the enzyme and sugars contained in the sugar liquid M3 and recovering the enzyme, and also has a function of supplying the sugar liquid M3 from which the enzyme has been recovered to the sugar liquid recovery section 6. The enzyme recovery section 5 has a storage section 50, a filtration section 51, an enzyme recovery tank 52, a transfer line 53, a transfer line 54, and a transfer line 55.

The storage section 50 is a portion for temporarily storing the sugar liquid M3. The storage section 50 is connected to the filtration section 51 via the transfer line 55. The sugar liquid M3 stored in the storage section 50 is supplied to the filtration section 51 via the transfer line 55 at a desired timing.

The filtration section 51 is preferably configured to separate the enzyme and sugars by a filtration separation method using an ultrafiltration membrane 511. That is, the enzyme recovery section 5 is preferably configured to have the ultrafiltration membrane 511 that separates the enzyme and sugars. As a result, the separation of the enzyme and sugars can be more favorably performed, and enzyme recovery can be more effectively performed.

The enzyme recovered in the filtration section 51 is transferred to the enzyme recovery tank 52 via the transfer line 53, and the sugar liquid M3 filtered in the filtration section 51 is transferred to the sugar liquid recovery section 6 via the transfer line 54.

The enzyme recovery tank 52 stores the separated enzyme. As a result, the enzyme can be stored, and a desired amount of the enzyme can be returned to the saccharification tank 1 at a desired timing.

### Sugar Liquid Recovery Section 6, Sugar Liquid Recovery Step

As shown in FIG. 1, the sugar liquid recovery section 6 has a sugar liquid recovery tank 61. The sugar liquid recovery tank 61 stores the filtered sugar liquid M3. As a result, a desired amount of the sugar liquid M3 can be taken out from the sugar liquid recovery tank 61 at a desired timing.

### Enzyme Return Line 7, Enzyme Return Step

As shown in FIG. 1, the enzyme return line 7 has a function of returning the enzyme recovered by the enzyme recovery section 5 to the saccharification tank 1. The enzyme return line 7 may be a flexible tube or a rigid pipe. One end of the enzyme return line 7 is connected to the enzyme recovery tank 52, and the other end is connected to the saccharification tank 1. Enzyme Detection Section 8, Enzyme Detection Step

The enzyme detection section 8 has a function of detecting the concentration of the enzyme contained in the saccharified liquid M2 in the saccharification tank 1. The detection method of the enzyme detection section 8 is not particularly limited, and examples thereof include an absorptiometric method, a fluorometric method, and a method using gel electrophoresis or the like. Known automatic spectrophotometers based on these methods can be applied to the present disclosure.

Examples of absorptiometric methods include ultraviolet spectrophotometry which is a method utilizing the absorption of ultraviolet light by a protein itself, the Bradford method and the WST method, which are methods utilizing the chemical binding between a protein and a chromogenic dye, and the Biuret method which is a method utilizing a copper ion chelate complex formed in the presence of a protein.

Examples of fluorometric methods include the Fluorescamine method, the o-Phthalaldehyde method, and the 3-(4-carboxybenzoyl) quinoline-2-carbox-aldehyde (CBQCA) method, which are methods using a reagent that emits fluorescence upon binding to primary amines in a protein, and the NanoOrange method, which is a method using a reagent that emits fluorescence upon binding to a surfactant that coats proteins.

Examples of methods using gel electrophoresis include polyacrylamide gel electrophoresis in which proteins are stained with a dye such as a fluorescent dye.

Among these, it is preferable to detect the enzyme concentration by the Bradford method. As a result, the concentration can be measured simply and accurately.

The enzyme detection section 8 is electrically connected to the control section 10, and information on the enzyme concentration detected by the enzyme detection section 8 is converted into an electric signal and transmitted to the control section 10.

### Extraction Line 9

An extraction line 9 connects the saccharification tank 1 and the enzyme detection section 8, and has a function of extracting the saccharified liquid M2 from the saccharification tank 1 and supplying the extracted saccharified liquid M2 to the enzyme detection section 8. The extraction line 9 may be a flexible tube or a rigid pipe. One end of the extraction line 9 is connected to the saccharification tank 1, and the other end is connected to the enzyme detection section 8.

Although not shown, the extraction line 9 is provided with a pump, a valve, and the like. As a result, the extraction line 9 exhibits a favorable liquid feeding function, and the saccharified liquid M2 can be supplied to the enzyme detection section 8 rapidly and stably. Control Section 10

As shown in FIG. 2, the control section 10 has a processor 101, a memory 102, and a communication section 103. The processor 101 is composed of, for example, a central processing unit (CPU), and reads and executes a program stored in the memory 102.

The memory 102 can be configured to have, for example, a volatile memory such as a RAM, a non-volatile memory such as a ROM, a detachable external storage device, or the like. Examples of programs stored in the memory 102 include a program related to the enzyme concentration measurement, a program related to the actuation timing of the discharge section, and the like.

The communication section 103 transmits and receives signals to and from the sections of the sugar liquid production system 100, such as the discharge section 2 and the enzyme detection section 8, and external devices using various external interfaces and the like.

Next, the actuation timing and the like of the discharge section 2 will be described.

The control section 10 controls the actuation of the discharge section 2 based on the detection result of the enzyme detection section 8 and controls the discharge operation of the saccharified liquid M2. In addition, the control section 10 controls the actuation of the discharge section 2 based on the temporal change in the enzyme concentration. Hereinafter, a specific description will be given.

When the mixed liquid M1 is fed into the saccharification tank 1, and the saccharification reaction between the raw material and the enzyme starts, a part of the saccharified liquid M2 in the saccharification tank 1 is supplied to the enzyme detection section 8 little by little. At this time, the discharge section 2 is closed, and the discharge of the saccharified liquid M2 via the discharge line 3 is not performed.

The enzyme detection section 8 transmits information on the enzyme concentration to the control section 10. The time required for one measurement by the automatic spectrophotometer applied to the enzyme detection section 8 is generally several minutes to about 10 minutes. The interval at which the enzyme detection section 8 transmits the concentration information to the control section 10 is not particularly limited, but is preferably an interval of 15 minutes or less, and more preferably an interval of 5 to 10 minutes.

FIG. 3 is an example of a graph in which the vertical axis represents the enzyme concentration detected by the enzyme detection section 8, and the horizontal axis represents the time (elapsed time) during which the saccharification reaction is performed. The temporal change in the enzyme concentration shows a tendency as described below.

First, the saccharification reaction starts at time (saccharification time "0 (h)" in the graph), and the saccharification reaction reaches saturation at time TA after about 1 hour. At this time, the enzyme concentration is about 45%.

During the period from time TA to time TB, the enzyme adsorbed on the solid M4 is desorbed from the solid M4, increasing the enzyme concentration, and at time TB, the enzyme concentration reaches about 95%. Thereafter, during the period until time TC, the enzyme is adsorbed again on the solid M4, decreasing the enzyme concentration, and the enzyme concentration reaches about 85% at time TC. After time TC, the concentration does not change sharply and remains flat.

Hereinafter, the period from time 0 to time TA, during which the enzyme concentration decreases, will be referred to as the "first concentration decrease period", the period from time TA to time TB, during which the enzyme concentration increases, will be referred to as the "first concentration increase period", and the period from time TB to time TC, during which the enzyme concentration decreases, will be referred to as the "second concentration decrease period".

At the peak of the first concentration increase period, after the completion of the saccharification reaction, the enzyme concentration is highest. It is most efficient to discharge the saccharified liquid M2 from the discharge section 2 and recover the enzyme at this time. The control section 10 discharges the saccharified liquid M2 from the discharge section 2 immediately after the end of the first concentration increase period of the enzyme concentration and the start of the second concentration decrease period. That is, the control section 10 detects information indicating a decrease in the enzyme concentration from time 0 (first concentration decrease period), detects information indicating an increase in the enzyme concentration (first concentration increase period), and then, upon the detection of information indicating a decrease in the enzyme concentration (second concentration decrease period), discharges the saccharified liquid M2 from the discharge section 2. As a result, enzyme recovery can be efficiently performed.

Hereinafter, examples of criteria for determining that the second concentration decrease period has started will be described.

### Pattern A

After the first concentration decrease period and the start of the first concentration increase period are detected, when the rate of decrease in the enzyme concentration (the amount of decrease in the concentration per unit time) exceeds a predetermined value, it is considered that the second concentration decrease period has started, and the saccharified liquid M2 is discharged from the discharge section 2.

### Pattern B

After the first concentration decrease period and the start of the first concentration increase period are detected, when a state in which the rate of decrease in the enzyme concentration (the amount of decrease in the concentration per unit time) is above a predetermined value has continued for a predetermined period of time, it is considered that the second concentration decrease period has started, and the saccharified liquid M2 is discharged from the discharge section 2.

### Pattern C

After the first concentration decrease period and the start of the first concentration increase period are detected, when the rate of increase in the enzyme concentration (the amount of increase in the concentration per unit time) falls below a predetermined value, it is considered that the first concentration increase period is about to end, indicating the start of the second concentration decrease period, and the saccharified liquid M2 is discharged from the discharge section 2.

### Pattern D

After the first concentration decrease period and the start of the first concentration increase period are detected, when a state in which the rate of increase in the enzyme concentration (the amount of increase in the concentration per unit time) is below a predetermined value has continued for a predetermined time, it is considered that the first concentration increase period is about to end, indicating the start of the second concentration decrease period, and the saccharified liquid M2 is discharged from the discharge section 2.

In addition to the above-described modes, the mode of the timing at which the saccharified liquid M2 is discharged from the discharge section 2 may also be such that the saccharified liquid M2 is discharged at a timing at which the fluctuation in the concentration of the enzyme contained in the saccharified liquid M2 decreases and the enzyme concentration becomes stable.

That is, in the example of the change in the enzyme concentration shown in FIG. 3, it can be seen that the enzyme remains at a high concentration in the saccharified liquid M2 even after time TC. Therefore, also by discharging the saccharified liquid M2 at an appropriate timing after time TC, enzyme recovery can be performed with high efficiency.

The timing for discharging the saccharified liquid M2 in this case is desirably a timing at which the fluctuation in the enzyme concentration converges within a predetermined range. For example, it is preferable that a timing at which the rate of fluctuation in the enzyme concentration falls within a range of -5% to +5% is detected and used as a trigger to discharge the saccharified liquid M2. In the example of FIG. 3, in terms of the saccharification time, the fluctuation in the enzyme concentration begins to decrease from approximately 20 h, and the enzyme concentration is stable during the time period from around 25 h to 30 h. Therefore, it is preferable to detect such a timing and discharge the saccharified liquid M2.

In order to measure the fluctuation in the enzyme concentration, calculation may be performed in the control section 10 by comparing multiple sets of concentration information transmitted from the enzyme detection section 8. That is, when the control section 10 calculates the difference or the rate of change in the enzyme concentration and determines that the calculation result falls within a predetermined range, it can be considered that the fluctuation in the enzyme concentration converges within the predetermined range. Then, it is preferable that the control section 10 actuates the discharge section 2 when such a determination is made.

In this way, the control section 10 controls the actuation of the discharge section 2 based on the detection result of the enzyme detection section 8 and controls the discharge operation. The control section 10 controls the actuation of the discharge section 2 based on the temporal change in the enzyme concentration. As a result, enzyme recovery can be efficiently performed. In particular, as described above, by discharging the saccharified liquid M2 before the enzyme is adsorbed again on the solid M4, enzyme recovery can be more efficiently performed.

As described above, the sugar liquid production system 100 includes a saccharification tank 1 in which a raw material containing cellulose is reacted with an enzyme to obtain a saccharified liquid M2 containing a sugar liquid M3 and a solid M4, a discharge line 3 that discharges the saccharified liquid M2 from the saccharification tank 1, a solid-liquid separation section 4 that separates the solid M4 from the saccharified liquid M2 discharged from the discharge line 3 to obtain the sugar liquid M3, an enzyme recovery section 5 that separates the enzyme and sugars contained in the sugar liquid M3 and recovers the enzyme, an enzyme return line 7 that returns the enzyme recovered by the enzyme recovery section 5 to the saccharification tank 1, an enzyme detection section 8 that detects a concentration of the enzyme contained in the saccharified liquid M2 in the saccharification tank 1, a discharge section 2 that performs a discharge operation of the saccharified liquid M2 from the saccharification tank 1, and a control section 10 that controls actuation of the discharge section 2 based on a detection result of the enzyme detection section 8 and controls the discharge operation. As a result, enzyme recovery can be efficiently performed.

In the present embodiment, the enzyme detection section 8 is configured to detect the enzyme concentration of the saccharified liquid M2 extracted from the saccharification tank 1 by the extraction line 9. However, the present disclosure is not limited thereto, and the enzyme detection section 8 may be configured to be installed in the saccharification tank 1.

In addition, the discharge section 2 is configured to have an on-off valve 21. However, the present disclosure is not limited thereto, and the discharge section 2 may be configured to suction and discharge the saccharified liquid M2 in the saccharification tank 1 by using a pump.

The control section 10 controls the actuation of the discharge section 2 based on the temporal change in the enzyme concentration. As a result, for example, by discharging the saccharified liquid M2 before the enzyme is adsorbed again on the solid M4, enzyme recovery can be more efficiently performed.

The enzyme recovery section 5 has an ultrafiltration membrane 511 that separates the enzyme and sugars. As a result, the separation of the enzyme and sugars can be more favorably performed, and enzyme recovery can be more effectively performed.

The enzyme recovery section 5 has an enzyme recovery tank 52 that stores the separated enzyme. As a result, the enzyme can be stored, and a desired amount of the enzyme can be returned to the saccharification tank 1 at a desired timing.

The enzyme return line 7 is provided between the enzyme recovery tank 52 and the saccharification tank 1. As a result, the enzyme recovered by the enzyme recovery section 5 can be returned from the enzyme recovery tank 52 to the saccharification tank 1.

The sugar liquid production system 100 includes an extraction line 9 installed between the saccharification tank 1 and the enzyme detection section 8, which extracts the saccharified liquid M2 from the saccharification tank 1 and supplies the extracted saccharified liquid M2 to the enzyme detection section 8. As a result, the saccharified liquid M2 can be extracted from the saccharification tank 1 and supplied to the enzyme detection section 8.

The saccharification tank 1 has a discharge port 12 that discharges the saccharified liquid M2, and the discharge section 2 has an on-off valve 21 provided at the discharge port 12. As a result, the saccharified liquid M2 can be discharged from the discharge port 12 by a simple method, that is, by controlling the opening/closing of the on-off valve 21. In addition, the timing of discharging the saccharified liquid M2 and stopping the discharge can be accurately controlled.

The sugar liquid production system of the present disclosure has been described above with reference to the embodiment shown in the drawings, but the present disclosure is not limited thereto, and each section constituting the sugar liquid production system can be replaced with any configuration capable of exhibiting the same function. In addition, any component may be added to the sugar liquid production system.

## Claims

1. A sugar liquid production system comprising:
a saccharification tank in which a raw material containing cellulose is reacted with an enzyme to obtain a saccharified liquid containing a sugar liquid and a solid;
a discharge line that discharges the saccharified liquid from the saccharification tank;
a solid-liquid separation section that separates the solid from the saccharified liquid discharged from the discharge line to obtain the sugar liquid;
an enzyme recovery section that separates the enzyme and sugars contained in the sugar liquid and recovers the enzyme;
an enzyme return line that returns the enzyme recovered by the enzyme recovery section to the saccharification tank;
an enzyme detection section that detects a concentration of the enzyme contained in the saccharified liquid in the saccharification tank;
a discharge section that performs a discharge operation of the saccharified liquid from the saccharification tank; and
a control section that controls actuation of the discharge section based on a detection result of the enzyme detection section and controls the discharge operation.

2. The sugar liquid production system according to claim 1, wherein the control section controls the actuation of the discharge section based on a temporal change in the concentration of the enzyme.

3. The sugar liquid production system according to claim 1, wherein the enzyme recovery section has an ultrafiltration membrane that separates the enzyme and the sugars.

4. The sugar liquid production system according to claim 1, wherein the enzyme recovery section has an enzyme recovery tank that stores the separated enzyme.

5. The sugar liquid production system according to claim 4, wherein the enzyme return line is installed between the enzyme recovery tank and the saccharification tank.

6. The sugar liquid production system according to claim 1, further comprising an extraction line installed between the saccharification tank and the enzyme detection section, wherein the extraction line extracts the saccharified liquid from the saccharification tank and supplies the extracted saccharified liquid to the enzyme detection section.

7. The sugar liquid production system according to claim 1, wherein
the saccharification tank has a discharge port that discharges the saccharified liquid, and
the discharge section has an on-off valve provided at the discharge port.
